# NEW EUROPEAN PATENT SPECIFICATION

(11) **EP 2 164 572 B2**
(45) Date of publication and mention of the opposition decision: **16.09.2015**
(45) Mention of the grant of the patent: 14.11.2012
(21) Application number: 08762655.2
(22) Date of filing: 19.05.2008
(51) Int. Cl.: A61P 25/18, A61K 31/495, A61K 31/4965

(54) **CARBAMOYL-CYCLOHEXANES FOR TREATING ACUTE MANIA**
CARBAMOYLCYCLOHEXANE ZUR BEHANDLUNG VON AKUTER MANIE
COMPOSITIONS PHARMACEUTIQUES ET MÉTHODE DE TRAITEMENT DU DÉLIRE AIGU

(30) Priority: 24.05.2007 HU 0700370
(43) Date of publication of application: 24.03.2010
(73) Proprietor: Richter Gedeon Nyrt., 1103 Budapest (HU)
(72) Inventor: LASZLOVSZKY, István, H-1111 Budapest (HU); NÉMETH, György, H-1021 Budapest (HU); ANDOR, György, H-1118 Budapest (HU)
(74) Representative: Hoffmann Eitle
(86) International application number: PCT/HU2008/000052
(87) International publication number: WO 2008/142463

(56) References cited:
- WO-A-2005/012266
- WO-A-2008/141135
- BOSE A ET AL: "The efficacy and safety of the novel antipsychotic cariprazine in acute exacerbation of schizophrenia", INTERNATIONAL JOURNAL OF NEUROPSYCHOPHARMACOLOGY, vol. 13, no. Suppl. 1, 2010, page 88, & 27TH COLLEGIUM INTERNATIONALE NEURO-PSYCHOPHARMACOLOGICUM CONGRESS; HONG KONG, PEOPLES R CHINA; JUNE 06 -10, 2010 ISSN: 1461-1457
- ERESHEFSKY L ET AL: "Phase I study of RGH-188 in schizophrenic patients", INTERNATIONAL JOURNAL OF NEUROPSYCHOPHARMACOLOGY, vol. 11, no. Suppl. 1, July 2008 (2008-07) , page 140, & 26TH COLLEGIUM INTERNATIONALE NEURO-PSYCHOPHARMACOLOGICUM CONGRESS (CINP); MUNICH, GERMANY; JULY 13 -17, 2008 ISSN: 1461-1457
- Poster entitled "The Efficacy and Tolerability of Cariprazine in Acute Mania Associated With Bipolar I Disorder: A Phase II Trial"
- M. KNESEVICH ET AL.: 'The efficacy and tolerability of cariprazine in acute mania associated with bipolar I disorder: a phase II trial' EUR. NEUROPSYCHOPHARMACOLOGY September 2009, pages 469 - 470
- J.R. CALABRESE ET AL.: 'Efficacy and safety of low-and high-dose cariprazine in patients with acute mania associated with bipolar I disorder' BIPOLAR DISORDERS no. 1, 2013, pages 104 - 155
- A. BOSE ET AL.: 'Cariprazine in the treatment of acute mania in bipolar disorder: a double-blind,placebo-controlled, phase III trial' SCHIZOPHRENIA 05 June 2012, pages 111 - 112
- Poster "Safety and tolerability of cariprazine in patients with acute bipolat mania: pooled analysis of 3 phase II/III pivotal studies

## Description

### FIELD OF THE INVENTION

The present invention is directed to a carbamoyl-cyclohexane derivative selected from trans-4-{2-[4-(2,3-dichlorophenyl)-piperazin-1-yl]-ethyl}-N,N-dimethylcarbamoyl-cyclohexylamine and/or salts and/or hydrates and/or solvates and/or polymorphs thereof, in a therapeutically effective amount for use in a method of treating acute mania by oral administration thereof, wherein the therapeutically effective amount is 3 to 12 mg/day.

### BACKGROUND OF THE INVENTION

Bipolar disorder in the United States affects 5.7 million adults or about 2.6% of the population 18 years of age and older in any given year and has considerable economic impact on our society. In a 1991 study conducted by the US National Institute of Mental Health, an estimated annual cost of $45 billion was attributed to bipolar disorder in the United States alone (Wyatt R J, Henter I., Soc. Psychiatry Psychiatr. Epidemiol., 30(5), 213-9, 1995). In the year 2000, this disorder ranked as the fifth leading cause of disability in adults between the ages of 15 and 44 (World Health Organization, World Health Report 2001, "Mental Health: New Understanding, New Hope." http://www.who.int/whr/2001/en/2001).

Bipolar disorder is a complex, chronic illness causing dramatic mood swings and unusual shifts in energy and behavior, ultimately resulting in functional impairments; it is associated with significant morbidity and mortality. It manifests itself as alterations in mood and energy from euphoria and excitability to depression and psychomotor retardation (Goodwin and Jamison, 1990 (Goodwin FK, Jamison KR. In: Manic-depressive illness. New York: Oxford University Press, 642-647, 1990), and is associated with significant morbidity and mortality. Suicide rates within this population are among the highest of all psychiatric illnesses (Müller-Oerlinghausen et al., Lancet, 359 (9302), 241-7, 2002). Bipolar disorder is treated in phases, with each phase presenting its own set of challenges to the treating physician. Bipolar mania accounts for one in seven psychiatric emergencies. Acute manic and mixed episodes are frequently associated with severe behavioral, physical, functional, and cognitive disturbances, all of which can have important personal and social consequences. For the most part, bipolar mania constitutes a medical emergency requiring a hospital admission to ensure the immediate safety of the patient or others and rapid symptomatic relief (Keck, British Medical Journal, 327 (7422), 1002-3, 2003).

A variety of pharmacological agents are currently available for the management of acute mania, including mood stabilizers, anticonvulsants, and antipsychotics, all of which can be used as monotherapy or in combination regimens. In recent years, the atypical antipsychotics (eg, olanzapine, risperidone, quetiapine, ziprasidone, aripiprazole) have been approved for mania in bipolar disorder. First- and second-generation antipsychotics are used in the acute setting in combination with mood stabilizers to achieve a more rapid control of symptoms in severely agitated patients whose treatment also necessitates hospitalization.

Compared to conventional agents, the side effect profile of atypical antipsychotics is more favorable. However, the atypicals have been associated with an increased risk of metabolic side effects, including body weight gain, dyslipidemia, glucose intolerance, and type II diabetes. Because of this increased risk, the FDA requires a warning label for diabetes on all atypical antipsychotics. Other side effects commonly associated with currently available treatment options for acute mania in bipolar patients include tremors, psychomotor slowing, cognitive impairment, exacerbation of agitation, nephrotoxicity, altered thyroid function, and sexual dysfunction.

Therefore, despite substantial advances in the pharmacological treatment of bipolar disorder, treatment needs are still not met by currently available therapies and only a low percentage of patients persistently benefit from treatment (Sachs, J. Clin. Psychopharmacol., 23 (3 Suppl 1), S2-8, 2003). A significant percentage of patients do not fully respond to these treatment options and continue to experience subthreshold symptoms and even relapse. This is attributed partly to the lack of efficacy of currently available medications, the production of intolerable side effects, and the increasing therapeutic costs (especially with use of combination regimens). These drawbacks limit their applicability and result or contribute to patient noncompliance. The optimum acute and long-term treatment strategies for acute bipolar mania are not yet established. More effective therapies with improved side effect profiles are still needed to enhance acute, as well as long-term, outcomes in these patients without the possibility of inducing depression or rapid cycling.

U.S. Patent Publication No. 2006/0229297 discloses (thio) carbamoyl-cyclohexane derivatives that are D₃ and D₂ dopamine receptor subtype preferring ligands, having the formula (I): 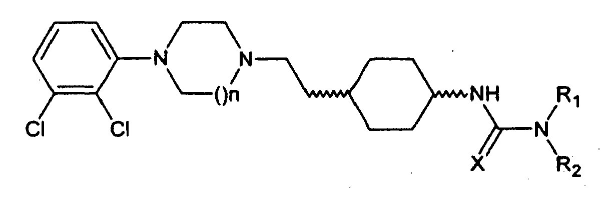 wherein R₁, R₂, X, and n are as defined therein.

Hungarian Patent Application No. P0700339 discloses salts of trans-4-{2-[4-(2,3-dichlorophenyl)-piperazin-1-yl]-ethyl}-N,N-dimethylcarbamoyl-cyclohexylamine. One particular compound disclosed therein is trans-4-{2-[4-(2,3-dichlorophenyl)-piperazin-1-yl]-ethyl}-N,N-dimethylcarbamoyl-cyclohexylamine hydrochloride, which is also known as trans-1{4-[2-[4-(2,3-dichlorophenyl)-piperazin-1-yl]-ethyl]-cyclohexyl}-3,3-dimethyl-urea hydrochloride, the structural formula for which is shown below:

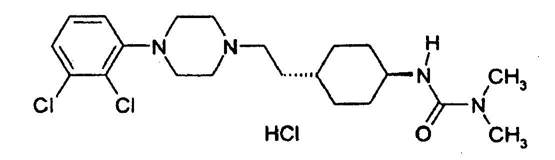

These (thio)-carbamoyl-cyclohexane derivatives are orally active and very potent dopamine D₃/D₂ receptor antagonists, which bind with significantly higher potency to D₃ than D₂ receptors.. The D₃ receptor antagonism is about one order of magnitude greater than the D₂ receptor antagonism, which is believed to counteract some of the extrapyramidal side effects produced by D₂ receptor antagonists. Some compounds e.g. trans-4-{2-[4-(2,3-dichlorophenyl)-piperazin-1-yl]-ethyl}-N,N-dimethylcarbamoyl-cyclohexylamine hydrochloride have another unique feature which is that in vivo it acts as a "dopamine system stabilizer." In this regard, it has preferential dopaminergic actions in the limbic regions and displays both (partial) agonist and antagonist activity on biosynthesis (and release) modulating presynaptic D₂ receptors depending on the functional status of the particular dopaminergic system.

In addition to the increased relative affinity for dopamine D₃ to D₂, trans-4-{2-[4-(2,3-dichlorophenyl)-piperazin-1-yl]-ethyl}-N,N-dimethylcarbamoyl-cyclohexylamine hydrochloride has a low potency at other receptor sites such as the 5-HT_{2C}, histamine H₁, and adrenergic receptor sites, which suggest a lower potential for side effects such as extrapyramidal symptoms (EPS) and body weight gain. Trans-4-{2-[4-(2,3-dichlorophenyl)-piperazin-1-yl]-ethyl}-N,N-dimethylcarbamoyl-cyclohexylamine hydrochloride also has considerable affinity for, and is a partial agonist at, the serotonin 5-HT_{1A} receptors, indicating that it may be effective in treating the depressive symptoms associated with bipolar disorder.

WO 2008/1411135 concerns solvate and crystalline forms of trans-1-{4-[2-[4-(2,3-dichlorophenyl)-piperazin-1-yl]-ethyl]-cyclohexyl}-3,3-dimethyl-urea hydrochloride and their use for treating conditions such as mania, acute mania, bipolar disorder as well as acute mania associated with bipolar disorder, wherein the dosage ranges from 1 to 500 mg or 0.1 to 100 mg daily, given in 1 to 4 doses, depending on the method of administration.

### SUMMARY OF THE INVENTION

The present invention is directed to a carbamoyl-cyclohexane derivative selected from trans-4-{2-[4-(2,3-dichlorophenyl)-piperazin-1-yl]-ethyl}-N,N-dimethylcarbamoyl-cyclohexylamine and/or salts and/or hydrates and/or solvates and/or polymorphs thereof, in a therapeutically effective amount for use in a method of treating acute mania by oral administration thereof, wherein the therapeutically effective amount is 3 to 12 mg/day.

### DETAILED DESCRIPTION OF THE INVENTION

The present invention is directed to a carbamoyl-cyclohexane derivative selected from trans-4-{2-[4-(2,3-dichlorophenyl)-piperazin-1-yl]-ethyl}-N,N-dimethylcarbamoyl-cyclohexylamine and/or salts and/or hydrates and/or solvates and/or polymorphs thereof, in a therapeutically effective amount for use in a method of treating acute mania by oral administration thereof, wherein the therapeutically effective amount is 3 to 12 mg/day.

Pharmaceutically acceptable salts include those obtained by reacting the main compound, functioning as a base with an inorganic or organic acid to form a salt, for example, salts of hydrochloric acid, sulfuric acid, phosphoric acid, methane sulfonic acid, camphor sulfonic acid, oxalic acid, maleic acid, succinic acid, citric acid, formic acid, hydrobromic acid, benzoic acid, tartaric acid, fumaric acid, salicylic acid, mandelic acid, and carbonic acid. Pharmaceutically acceptable salts also include those in which the main compound functions as an acid and is reacted with an appropriate base to form, e.g., sodium, potassium, calcium, magnesium, ammonium, and choline salts. Those skilled in the art will further recognize that acid addition salts of the claimed compounds may be prepared by reaction of the compounds with the appropriate inorganic or organic acid via any of a number of known methods. Alternatively, alkali and alkaline earth metal salts can be prepared by reacting the compounds of the invention with the appropriate base via a variety of known methods.

The following are further examples of acid salts that can be obtained by reaction with inorganic or organic acids: acetates, adipates, alginates, citrates, aspartates, benzoates, benzenesulfonates, bisulfates, butyrates, camphorates, digluconates, cyclopentanepropionates, dodecylsulfates, ethanesulfonates, glucoheptanoates, glycerophosphates, hemisulfates, heptanoates, hexanoates, fumarates, hydrobromides, hydroiodides, 2-hydroxy-ethanesulfonates, lactates, maleates, methanesulfonates, nicotinates, 2-naphthalenesulfonates, oxalates, palmoates, pectinates, persulfates, 3-phenylpropionates, picrates, pivalates, propionates, succinates, tartrates, thiocyanates, tosylates, mesylates and undecanoates.

In one embodiment, the pharmaceutically acceptable salt is a hydrochloride salt.

The compound useful in the present invention can exist in different polymorphic forms. As known in the art, polymorphism is an ability of a compound to crystallize as more than one distinct crystalline or "polymorphic" species. A polymorph is a solid crystalline phase of a compound with at least two different arrangements or polymorphic forms of that compound molecule in the solid state. Polymorphic forms of any given compound are defined by the same chemical formula or composition and are as distinct in chemical structure as crystalline structures of two different chemical compounds. The use of such polymorphs is within the scope of the present invention.

The compound useful in the present invention can exist in different solvate forms. Solvates of the compounds of the invention may also form when solvent molecules are incorporated into the crystalline lattice structure of the compound molecule during the crystallization process. For example, suitable solvates include hydrates, e.g., monohydrates, dihydrates, sesquihydrates, and hemihydrates. The use of such solvates is within the scope of the present invention.

The active ingredient is administered in an amount of 3 - 12 mg/day.

In exemplary embodiments, the active ingredient is administered in an amount of 3 mg, about 4.5 mg, about 6 mg, about 9 mg or 12 mg, for example, in an amount of 3 mg, about 6 mg, about 9 mg or 12 mg.

The desired dose may be administered as one or more daily sub dose(s) administered at appropriate time intervals throughout the day, or alternatively, in a single dose, for example, for morning or evening administration. For example, the daily dosage may be divided into one, into two, into three, or into four divided daily doses.

The duration of the treatment may be decades, years, months, weeks, or days, as long as the benefits persist.

In another embodiment, the present invention relates to a compound for use in a method of treating acute mania by administering to a patient in need thereof compound for use in a 3 mg trans-4-{2-[4-(2,3-dichlorophenyl)-piperazin-1-yl]-ethyl}-N,N-dimethylcarbamoyl-cyclohexylamine or a pharmaceutically acceptable salt thereof.

In a further embodiment, the present invention relates to a compound for use in a method of treating acute mania by administering to a patient in need thereof about 4.5 mg trans-4-{2-[4-(2,3-dichlorophenyl)-piperazin-1-yl]-ethyl}-N,N-dimethylcarbamoyl-cyclohexylamine or a pharmaceutically acceptable salt thereof.

In yet another embodiment, the present invention relates to a compound for use in a method of treating acute mania by administering to a patient in need thereof about 6 mg trans-4-{2-[4-(2,3-dichlorophenyl)-piperazin-1-yl]-ethyl}-N,N-dimethylcarbamoyl-cyclohexylamine or a pharmaceutically acceptable salt thereof.

In another embodiment, the present invention relates to a compound for use in a method of treating acute mania by administering to a patient in need thereof about 9 mg trans-4-{2-[4-(2,3-dichlorophenyl)-piperazin-1-yl]-ethyl}-N,N-dimethylcarbamoyl-cyclohexylamine or a pharmaceutically acceptable salt thereof.

In yet another embodiment, the present invention relates to a compound for use in a method of treating acute mania by administering to a patient in need thereof compound for use in a 12 mg trans-4-{2-[4-(2,3-dichlorophenyl)-piperazin-1-yl]-ethyl}-N,N-dimethylcarbamoyl-cyclohexylamine or a pharmaceutically acceptable salt thereof.

In additional embodiments, the active ingredient administered is trans-4-{2-[4-(2,3-dichlorophenyl)-piperazin-1-yl]-ethyl}-N,N-dimethylcarbamoyl-cyclohexylamine hydrochloride.

In one embodiment, the active ingredient is administered in one or two divided daily doses.

In one embodiment, the administration of the active ingredient provides therapeutic effects in the treatment of acute mania associated with bipolar disorder, e.g., acute mania associated with bipolar I disorder.

The active ingredient can be administered alone or as an active ingredient of a pharmaceutical composition.

Numerous standard references are available that describe procedures for preparing various formulations suitable for administering the compound according to the invention. Examples of potential formulations and preparations are contained, for example, in the Handbook of Pharmaceutical Excipients, American Pharmaceutical Association (current edition); Pharmaceutical Dosage Forms: Tablets (Lieberman, Lachman and Schwartz, editors) current edition, published by Marcel Dekker, Inc., as well as Remington's Pharmaceutical Sciences (Arthur Osol, editor), 1553-1593 (current edition)

The active ingredient is formulated for oral administration.

To prepare such pharmaceutical dosage forms, the active ingredient, is intimately admixed with a pharmaceutical carrier according to conventional pharmaceutical compounding techniques. The carrier may take a wide variety of forms depending on the form of preparation desired for administration.

In preparing the compositions in oral dosage form, any of the usual pharmaceutical media may be employed. Thus, for liquid oral preparations, such as, for example, suspensions, elixirs and solutions, suitable carriers and additives include water, glycols, oils, alcohols, flavoring agents, preservatives, coloring agents and the like. For solid oral preparations such as, for example, powders, capsules and tablets, suitable carriers and additives include starches, sugars, diluents, granulating agents, lubricants, binders, disintegrating agents and the like. Due to their ease in administration, tablets and capsules represent the most advantageous oral dosage unit form. If desired, tablets may be sugar coated or enteric coated by standard techniques.

In some applications, it may be advantageous to utilize the active agent in a "vectorized" form, such as by encapsulation of the active agent in a liposome or other encapsulant medium, or by fixation of the active agent, e.g., by covalent bonding, chelation, or associative coordination, on a suitable biomolecule, such as those selected from proteins, lipoproteins, glycoproteins, and polysaccharides.

Formulations used in the present invention for oral administration may be presented as discrete units such as capsules, cachets, tablets, or lozenges, each containing a predetermined amount of the active ingredient as a powder or granules. Optionally, a suspension in an aqueous liquor or a non-aqueous liquid may be employed, such as a syrup, an elixir, an emulsion, or a draught.

A tablet may be made by compression or molding, or wet granulation, optionally with one or more accessory ingredients. Compressed tablets may be prepared by compressing in a suitable machine, with the active compound being in a free-flowing form such as a powder or granules which optionally is mixed with, for example, a binder, disintegrant, lubricant, inert diluent, surface active agent, or discharging agent. Molded tablets comprised of a mixture of the powdered active compound with a suitable carrier may be made by molding in a suitable machine.

A syrup may be made by adding the active compound to a concentrated aqueous solution of a sugar, for example sucrose, to which may also be added any accessory ingredient(s). Such accessory ingredient(s) may include flavorings, suitable preservative, agents to retard crystallization of the sugar, and agents to increase the solubility of any other ingredient, such as a polyhydroxy alcohol, for example glycerol or sorbitol.

In addition to the aforementioned ingredients, formulations of this invention may further include one or more accessory ingredient(s) selected from diluents, buffers, flavoring agents, binders, disintegrants, surface active agents, thickeners, lubricants, preservatives (including antioxidants), and the like.

The formulations of the present invention can have immediate release, sustained release, delayed-onset release or any other release profile known to one skilled in the art.

In one embodiment of the present invention, trans-4-{2-[4-(2,3-dichlorophenyl)-piperazin-1-yl]-ethyl}-N,N-dimethylcarbamoyl-cyclohexylamine or a pharmaceutically acceptable salt thereof is administered as an adjunctive treatment to or in combination with one or more additional therapeutic agents.

### Definitions

The term "pharmaceutically acceptable" means biologically or pharmacologically compatible for *in vivo* use in animals or humans, and preferably means approved by a regulatory agency of the Federal or a state government or listed in the U.S. Pharmacopeia or other generally recognized pharmacopeia for use in animals, and more particularly in humans.

The terms "treat," "treatment," and "treating" refer to one or more of the following:
(a) relieving or alleviating at least one symptom of a disorder in a subject, including for example, allergic and inflammatory disorders, such as asthma and COPD;
(b) relieving or alleviating the intensity and/or duration of a manifestation of a disorder experienced by a subject including, but not limited to, those that are in response to a given stimulus (e.g., pressure, tissue injury, cold temperature, etc.);
(c) arresting, delaying the onset (i.e., the period prior to clinical manifestation of a disorder) and/or reducing the risk of developing or worsening a disorder.

An "effective amount" means the amount of an active ingredient that, when administered to a patient (e.g., a mammal) for treating a disease or disorder, is sufficient to effect such treatment for the disease or disorder, or an amount that is sufficient for modulating a dopamine receptor (e.g., the dopamine D₂ and/or dopamine D₃ receptor) to achieve the objectives of the invention. The "effective amount" will vary depending on the compound, the disease and its severity and the age, weight, responsiveness, etc., of the patient to be treated.

A subject or patient in whom administration of the therapeutic compound is an effective therapeutic regimen for a disease or disorder is preferably a human, but can be any animal, including a laboratory animal in the context of a trial or screening or activity experiment. Thus, as can be readily appreciated by one of ordinary skill in the art, the compounds and compositions of the present invention are particularly suited to administration to any animal, particularly a mammal, and including, but by no means limited to, humans, domestic animals, such as feline or canine subjects, farm animals, such as but not limited to bovine, equine, caprine, ovine, and porcine subjects, wild animals (whether in the wild or in a zoological garden), research animals, such as mice, rats, rabbits, goats, sheep, pigs, dogs, cats, etc., avian species, such as chickens, turkeys, songbirds, etc., i.e., for veterinary medical use.

The term "about" or "approximately" means within an acceptable error range for the particular value as determined by one of ordinary skill in the art, which will depend in part on how the value is measured or determined, i.e., the limitations of the measurement system. For example, "about" can mean within 1 or more than 1 standard deviations, per practice in the art. Alternatively, "about" with respect to the compositions can mean plus or minus a range of up to 20%, preferably up to 10%, more preferably up to 5%.

### EXAMPLES

### EXAMPLE 1

This clinical study will be conducted as a multicenter, randomized, double-blind, placebo-controlled, parallel-group, flexible-dose study. A total of approximately 240 inpatients patients will be selected who meet criteria that include:
1. Men and women 18 to 65 years of age at Visit 1
2. Patients must meet DSM-IV-TR criteria for bipolar I disorder (confirmed by the administration of the Structured Clinical Interview (SCID)), acute manic or mixed episode type with or without psychotic symptoms. Comorbid diagnoses such as conduct disorder, obsessive-compulsive disorder, anxiety disorders, and substance abuse are allowed
3. Patients must have a YMRS total score ≥ 20 at Visit 1 and Visit 2 and a score of at least 4 on two of the following YMRS items: Irritability, Speech, Content, and Disruptive/Aggressive Behavior

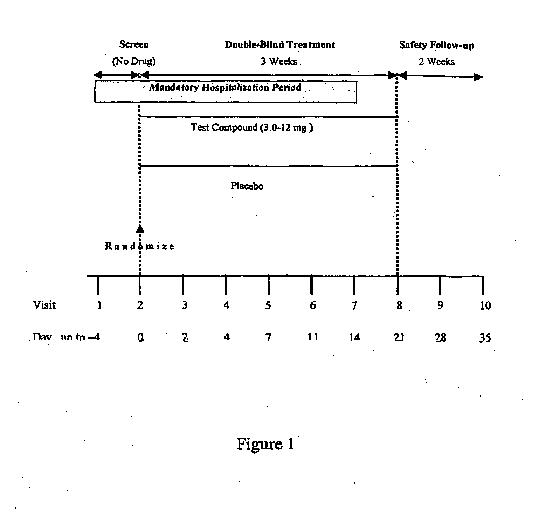

This study will be 5 weeks in duration; 3-weeks of double-blind treatment and 2-weeks of safety follow-up. Patients will start hospitalization during the screening phase. A no-drug washout period of up to 4 days will precede randomization. Following the washout period, patients who continue to meet all eligibility criteria will be assigned a randomization number at Visit 2 and dispensed the corresponding blister pack of double-blind study medication for Week 1 of double-blind treatment. The study design is shown on Figure 1.

Following the completion of 3 weeks of double-blind treatment or premature discontinuation during the double-blind phase, patients will be followed up for safety assessments for an additional 2 weeks. During this period, patients will receive treatment as usual (TAU) at the discretion of the Investigator.

### Dosing Regimen

All patients meeting the eligibility criteria will be randomized (1:1 ratio) into one of two treatment groups:
(I) placebo,
(II) 3.0 - 12.0 mg trans-4-{2-[4-(2,3-dichlorophenyl)-piperazin-1-yl]-ethyl}-N,N-dimethylcarbamoyl-cyclohexylamine hydrochloride.

Patients will be supplied with identically appearing capsules containing 1.5 mg or 3.0 mg, of trans-4-{2-[4-(2,3-dichlorophenyl)-piperazin-1-yl]-ethyl}-N,N-dimethylcarbamoyl-cyclohexylamine hydrochloride or placebo.

All study medication will be dispensed in blister packs, one for each week. Each card will contain 40 capsules arranged in 10 columns and 4 rows, adequate for the 7 days of the week plus 3 extra days. The configuration of the blister pack is provided in Table 1. All study drugs will be administered as a single daily dose at bedtime. The dosing can be switched to morning if there are tolerability problems; however, any switch must allow at least 24 hours between two consecutive doses.

**Table 1: Double-Blind Study Dosing Regimen**

| | **Treatment Group:** | | | | |
|---|---|---|---|---|---|
| | **Trans-4-{2-[4-(2,3-dichlorophenyl)-piperazin-1-yl]-ethyl}-N,N-dimethylcarbamoyl-cyclohexylamine hydrochloride** | | | | |
| | **Day 0** | **Day 1** | **Day 2** | **Day 3** | **Day 4-21** |
| **Row 1** | 1.5 mg | 3 mg | 3 mg | 3 mg | 3 mg |
| **Row 2** | 0 | 0 | 3 mg | 3 mg | 3 mg |
| **Row 3** | 0 | 0 | 0 | 3 mg | 3 mg |
| **Row 4** | 0 | 0 | 0 | 0 | 3 mg |
| | **Treatment Group: Placebo** | | | | |
| **Row 1** | 0 | 0 | 0 | 0 | 0 |
| **Row 2** | 0 | 0 | 0 | 0 | 0 |
| **Row 3** | 0 | 0 | 0 | 0 | 0 |
| **Row 4** | 0 | 0 | 0 | 0 | 0 |

On day 0 and day 1, all patients will be administered one capsule from Row 1 of the blister pack. On day 2, the dose can be increased to two capsules (Rows 1 and 2), if response is not adequate and there are no tolerability problems. On day 3, the dose can be increased by one capsule to three capsules (Rows 1, 2, and 3) depending on response and tolerability. Starting on day 4, the dose can be increased by one capsule to a maximum of 4 capsules (Rows 1, 2, 3 and 4) depending on the response and tolerability. Any dose increases will be made in increments of one capsule.

Following a dose increase, if there are any tolerability problems, the dose can be decreased at any time to the prior level by eliminating the last row. For example, Row 4 can be eliminated if the patient was on four capsules; Row 3 can be eliminated if the patient was on three capsules; Row 2 can be eliminated if the patient was on two capsules. Any decreases in the dosing regimen will be carried out in decrements of one capsule. Alternatively, the dose can be skipped for 1 to 2 days if there are tolerability problems. However, frequent switching will not be allowed.

### Patient Evaluations

The patient evaluation schedule is shown in Table 2.

**Table 2: Evaluation Schedule**

| | **Screening** | **Baseline** | **Double-Blind Phase** | | | | | | **Safety Follow-up** | |
|---|---|---|---|---|---|---|---|---|---|---|
| | Visit 1 | Visit 2 | Visit 3 | Visit 4 | Visit 5 | Visit 6 | Visit 7¹ | Visit 8² | Visit 9² | Visit 10^{2,3} |
| Study Day | up to -4 | 0 | 2 | 4 | 7 | 11 | 14 | 21 | 28 | 35 |

| | | | | | | | | | | |
|---|---|---|---|---|---|---|---|---|---|---|
| 1: Early discharge procedures will be performed on Day 14 (Visit 7) or any day before day 21 (Visit 8). If criteria are met, the patient will discharge and complete the remained of the study as an outpatient 2: Performed for all patients including those prematurely discontinued after randomization (Visit 2). 3: Clinical findings upon termination will be followed-up until the condition returns to pretrial status or can be explained as unrelated to study medication. If necessary, a follow-up visit will be scheduled. | | | | | | | | | | |

The descriptions of the procedures to be performed at each visit are provided below. Visits 8, 9 and 10 may be conducted up to 3 days before or after the scheduled visit.

### Screening (Visit 1)

A review of inclusion/exclusion criteria will be conducted to determine the patient's eligibility for enrollment. Study procedures will be reviewed with the patient, and documentation of informed consent will be obtained. Upon signing the informed consent form, patients will be assigned a unique PID in sequential order.
- Medical and surgical, neurological, and psychiatric histories
- Obtain prior and concomitant medication history
- Physical examination (to be performed by a study physician)
- Assess vital-signs (temperature, height, weight, blood pressure (including orthostatic value), and radial pulse (including orthostatic value)
- Peform 12-lead ECG
- Collect blood and urine samples for clinical laboratory determinations (hematology, fasting serum chemistry, urinalysis, prolactin, HbA_{1c}, TSH, Free-T4, β-HCG [pregnancy test in women of childbearing potential])
- Collect blood sample for concentration of lithium (for patients taking lithium prior to Screening)
- Collect urine sample for drug screen
- Collect sample for blood alcohol level
- Conduct SCID interview
- Administer YMRS, CGI-S, and MADRS
- Review inclusion/exclusion criteria
- Record prior and concomitant medications
- Determine washout eligibility and duration

A washout period of up to 4 days may be required for patients who are receiving or are suspected of having received any prohibited medications. A 3-day washout period will also be required for patients who have been intoxicated with alcohol or who have tested positive for tetrahydrocannabinol/cannaboids. A 4-day washout period will be required for patients who have taken or are suspected of taking other illicit drugs.

### Baseline (Visit 2)

This visit is to be conducted within 4 days after Visit 1. The inclusion/exclusion criteria will be reviewed; and, if the patient is eligible to enter the study, a randomization number will be assigned. Study procedures will then be reviewed with the patient.
- Assess vital-signs (temperature, weight, blood pressure (including orthostatic value), and radial pulse (including orthostatic value)
- Establish that benzodiazepines have not been taken by the patient for at least 8 hours before behavioral assessment
- Administer YMRS, CGI-S, Positive and Negative Syndrome Scale (PANSS), and MADRS
- Administer BARS, AIMS, and SAS
- Review inclusion/exclusion criteria
- Review of AEs since the last visit
- Record concomitant medications
- Assign randomization number if the patient meets eligibility criteria
- Dispense double-blind study medication

### Visits 3-7

- Assess vital-signs (temperature, weight, blood pressure (including orthostatic value), and radial pulse (including orthostatic value)
- Collect blood and urine samples for clinical laboratory determinations (hematology, fasting serum chemistry, and urinalysis will be collected at Visit 6 only)
- Perform 12-lead ECG (at Visit 6 only)
- Administer YMRS, CGI-S, and CGI-I
- Administer MADRS (at Visits 5 and 7 only)
- Administer PANSS (at Visit 7 only)
- Administer BARS, AIMS, and SAS
- Review of AEs since the last visit
- Record concomitant medications
- Collect all unused double-blind study medication dispensed at previous visit (Visit 5, 7 and 8 only)
- Dispense study medication (Visit 5 and 7 only)

### Visit 8 or Early Termination

- Physical examination (to be conducted by a study physician)
- Assess vital-signs (temperature, weight, blood pressure [including orthostatic value], and radial pulse [including orthostatic value])
- Collect urine drug screen (only for outpatients discharged between Visits 7 and 8)
- Perform 12-lead ECG
- Collect blood and urine samples for clinical laboratory determinations (hematology, fasting serum chemistry, urinalysis, prolactin, TSH, free T4) and a β-HCG pregnancy test in women of childbearing potential
- Administer YMRS, CGI-I, and CGI-S
- Administer MADRS and PANSS
- Administer BARS, AIMS, and SAS
- Review of AEs since the last visit
- Record concomitant medications
- Collect all unused double-blind study medication dispensed at previous visit

### Visits 9 and 10

Before each patient is released from the study, either at the conclusion of the study or upon premature termination, he or she will be cross-titrated and stabilized on an appropriate medication as deemed necessary by the Investigator. Two safety follow-up visits, Visits 9 and 10, will be scheduled for all patients approximately 7 and 14 days after the last dose of study medication. The following evaluations will be performed:
- Assess vital-signs (temperature, weight, blood pressure (including orthostatic value)
- Review of AEs since the last visit
- Record concomitant medications

Other evaluations, such as laboratory assessments, UDS, and ECG, will only be repeated at these visits if there was an abnormal finding obtained at the most recent evaluation or if additional information is clinically necessary to appropriately follow up and manage an adverse experience.

Any clinical findings obtained in the final assessments performed at Visit 10 or at premature discontinuation for any reason, including clinically significant laboratory abnormalities, will be followed until the condition returns to pre-study status or can be explained as being unrelated to study medication. A follow-up visit, if one is necessary, will be scheduled within 30 days of termination.

### Efficacy Measurements

### Primary Efficacy Assessment

### Young Mania Rating Scale (YMRS)

The YMRS (see, e.g., Young et al., Br. J. Psychiatry, 133, 429-35, 1978) is an 11-item scale that assesses manic symptoms based on the patient's perception of his or her condition over the previous 48 hours, as well as the physician's clinical observations during the interview. The 11 items are elevated mood, increased motor activity-energy, sexual interest, sleep, irritability, rate and amount of speech, language-thought disorder, content, disruptive-aggressive behavior, appearance, and insight. The severity of the abnormality is rated on a five-point (0-4) or nine-point (0-8) scale; scoring between listed points is encouraged. Possible scores range from 0 to 60. This scale will be administered by a trained rater with expertise in evaluating manic patients. Assessments and ratings should be made by the same rater at approximately the same time of day.

### Secondary Efficacy Assessment

### Clinical Global Impressions-Severity (CGI-S)

The CGI-S (see, e.g., Guy ECDEU Assessment Manual for Psychopharmacology. Rockville, Md: US Department of Health, Education, and Welfare, 218-22, 1976. Publication ADM 76-338) is a seven-point scale that measures the overall severity of the illness in comparison to the severity of other patients the physician has observed. For patients discharged between Day 14 through Day 21, a CGI-S is required to be conducted within 24 hours prior to discharge from the hospital. This assessment will be made by a psychiatrist.

### Additional Efficacy Assessments

### Clinical Global Impressions-Improvement (CGI-I)

The Clinical Global Impressions-Improvement (CGI-I) (see, e.g., Guy ECDEU Assessment Manual for Psychopharmacology. Rockville, Md: US Department of Health, Education, and Welfare, 218-22, 1976. Publication ADM 76-338) is a seven-point scale that measures the change from Baseline (Visit 2) in the overall severity of illness for the individual patient. The CGI-I will be assessed by a psychiatrist.

### Montgomery-Asberg Depression Rating Scale (MADRS)

The MADRS (see e.g., Montgomery and Asberg, Br. J. Psychiatry, 134, 382-9, 1979) is a clinician-rated scale that evaluates the patient's depressive symptomatology during the past week. Patients are to be rated on 10 items assessing feelings of sadness, lassitude, pessimism, inner tension, suicidality, reduced sleep or appetite, difficulty in concentration, and lack of interest. Each item will be scored on a seven-point scale with a score of 0 reflecting no symptoms and a score of 6 reflecting symptoms of maximum severity. This scale will be administered by a trained rater with adequate experience in the assessment of the patient's depressive symptomology.

### Positive and Negative Syndrome Scale (PANSS)

The PANSS (see, e.g., Kay et al. Schizophr. Bull., 13, 261-76, 1987) is a 30-item rating scale that was specifically developed to assess both the positive and negative symptom syndromes of patients with schizophrenia. The PANSS Total Score is rated based on a structured clinical interview with the patient and supporting clinical information obtained from family, hospital staff, or other reliable informants. Each item is scored on a seven-point (1-7) continuum and provides scores in nine clinical domains, including a positive syndrome, a negative syndrome, depression, a composite index, and general psychopathology._This scale will be administered by a trained, experienced psychiatric rater with expertise in the assessment of patients with bipolar disorder and schizophrenia.

It is anticipated that the aforementioned treatment regime with trans-4-{2-[4-(2,3-dichlorophenyl)-piperazin-1-yl]-ethyl}-N,N-dimethylcarbamoyl-cyclohexylamine hydrochloride will show significant surprising effectiveness in the treatment of acute mania, e.g., acute mania associated with bipolar I disorder, when compared to patients treated with placebo.

The present invention is not to be limited in scope by the specific embodiments described herein. Indeed, various modifications of the invention in addition to those described herein will become apparent to those skilled in the art from the foregoing description and the accompanying figures. Such modifications are intended to fall within the scope of the appended claims. It is further to be understood that all values are approximate, and are provided for description.

## Claims

1. A carbamoyl-cyclohexane derivative selected from trans-4-{2-[4-(2,3-dichlorophenyl)-piperazin-1-yl]-ethyl}-N,N-dimethylcarbamoyl-cyclohexylamine and/or salts and/or hydrates and/or solvates and/or polymorphs thereof, in a therapeutically effective amount for use in a method of treating acute mania by oral administration thereof, wherein the therapeutically effective amount is 3 to 12 mg/day.

2. Carbamoyl-cyclohexane derivative for use according to claim 1, wherein the carbamoyl-cyclohexane derivative is trans-4-{2-[4-(2,3-dichlorophenyl)-piperazin-1-yl]-ethyl}-N,N-dimethylcarbamoyl-cyclohexylamine hydrochloride and/or hydrates and/or solvates and/or polymorphs thereof.

3. Carbamoyl-cyclohexane derivative for use according to claim 1 or 2 **characterized by** that the therapeutically effective amount is 3.0 mg/day.

4. Carbamoyl-cyclohexane derivative for use according to claim 1 or 2 **characterized by** that the therapeutically effective amount of is 4.5 mg/day.

5. Carbamoyl-cyclohexane derivative for use according to claim 1 or 2 **characterized by** that the therapeutically effective amount is 6 mg/day.

6. Carbamoyl-cyclohexane derivative for use according to claim 1 or 2 **characterized by** that the therapeutically effective amount of 9 mg/day.

7. Carbamoyl-cyclohexane derivative for use according to claim 1 or 2 **characterized by** that the therapeutically effective amount is 12 mg/day.

8. Carbamoyl-cyclohexane derivative for use according to any one of claims 1-7 **characterized by** that the therapeutically effective amount is divided into one, two, three or four daily doses.

9. Carbamoyl-cyclohexane derivative for use according to any one of claims 1-8 **characterized by** that the acute mania is associated with bipolar disorder.

10. Carbamoyl-cyclohexane derivative for use according to claim 9 **characterized by** that the bipolar disorder is bipolar I disorder.

11. Carbamoyl-cyclohexane derivative for use according to claim 9 **characterized by** that the bipolar disorder is bipolar II disorder.

12. Carbamoyl-cyclohexane derivative for use according to claim 9 **characterized by** that the bipolar disorder is cyclothymic disorder.

13. Carbamoyl-cyclohexane derivative for use according to any one of claims 1-7 **characterized by** that the acute mania is associated with acute manic and mixed episodes.

## Patentansprüche

1. Carbamoylcyclohexan-Derivat ausgewählt aus Trans-4-{2-[4-(2,3-dichlorphenyl)-piperazin-1-yl]-ethyl}-N,N-dimethylcarbamoylcyclohexylamin und/oder Salzen und/oder Hydraten und/oder Solvaten und/oder Polymorphen davon, in einer therapeutisch wirksamen Menge zur Verwendung in einem Verfahren zur Behandlung akuter Manie durch orale Verabreichung, worin die therapeutisch wirksame Menge 3 bis 12 mg/Tag beträgt.

2. Carbamoylcyclohexan-Derivat zur Verwendung gemäß Anspruch 1, worin das Carbamoylcyclohexan-Derivat Trans-4-{2-[4-(2,3-dichlorphenyl)-piperazin-1-yl]-ethyl}-N,N-dimethylcarbamoylcyclohexylaminhydrochlorid und/oder Hydrate und/oder Salze und/oder Polymorphe davon ist.

3. Carbamoylcyclohexan-Derivat zur Verwendung gemäß Anspruch 1 oder 2, **dadurch gekennzeichnet, dass** die therapeutisch wirksame Menge 3,0 mg/Tag beträgt.

4. Carbamoylcyclohexan-Derivat zur Verwendung gemäß Anspruch 1 oder 2, **dadurch gekennzeichnet, dass** die therapeutisch wirksame Menge 4,5 mg/Tag beträgt.

5. Carbamoylcyclohexan-Derivat zur Verwendung gemäß Anspruch 1 oder 2, **dadurch gekennzeichnet, dass** die therapeutisch wirksame Menge 6 mg/Tag beträgt.

6. Carbamoylcyclohexan-Derivat zur Verwendung gemäß Anspruch 1 oder 2, **dadurch gekennzeichnet, dass** die therapeutisch wirksame Menge 9 mg/Tag beträgt.

7. Carbamoylcyclohexan-Derivat zur Verwendung gemäß Anspruch 1 oder 2, **dadurch gekennzeichnet, dass** die therapeutisch wirksame Menge 12 mg/Tag beträgt.

8. Carbamoylcyclohexan-Derivat zur Verwendung gemäß einem der Ansprüche 1 bis 7, **dadurch gekennzeichnet, dass** die therapeutisch wirksame Menge in eine, zwei, drei oder vier täglichen Dosen unterteilt ist.

9. Carbamoylcyclohexan-Derivat zur Verwendung gemäß einem der Ansprüche 1 bis 8, **dadurch gekennzeichnet, dass** die akute Manie mit bipolarer Störung in Verbindung steht.

10. Carbamoylcyclohexan-Derivat zur Verwendung gemäß Anspruch 9, **dadurch gekennzeichnet, dass** die bipolare Störung Bipolar-I-Störung ist.

11. Carbamoylcyclohexan-Derivat zur Verwendung gemäß Anspruch 9, **dadurch gekennzeichnet, dass** die bipolare Störung Bipolar-II-Störung ist.

12. Carbamoylcyclohexan-Derivat zur Verwendung gemäß Anspruch 9, **dadurch gekennzeichnet, dass** die bipolare Störung Cyclothymia ist.

13. Carbamoylcyclohexan-Derivat zur Verwendung gemäß irgendeinem der Ansprüche 1 bis 7, **dadurch gekennzeichnet, dass** die akute Manie mit akuter Manie und gemischten Zuständen in Verbindung steht.

## Revendications

1. Dérivé de carbamoyl-cyclohexane choisi parmi la trans-4-{2-[4-(2,3-dichlorophényl)-pipérazin-l-yl]-éthyl}-N,N-diméthylcarbamoyl-cyclohexylamine et/ou ses sels et/ou hydrates et/ou solvates et/ou polymorphes de celle-ci, en une quantité efficace du point de vue thérapeutique pour une utilisation dans une méthode de traitement de la manie aiguë par son administration orale, dans laquelle la quantité efficace du point de vue thérapeutique est de 3 à 12 mg/jour.

2. Dérivé de carbamoyl-cyclohexane pour une utilisation selon la revendication 1, dans lequel le dérivé de carbamoyl-cyclohexane est le chlorhydrate de trans-4-{2-[4-(2,3-dichlorophényl)-pipérazin-1-yl]-éthyl}-N,N-diméthylcarbamoyl-cyclohexylamine et/ou ses hydrates et/ou solvates et/ou polymorphes.

3. Dérivé de carbamoyl-cyclohexane pour une utilisation selon la revendication 1 ou 2, **caractérisé en ce que** la quantité efficace du point de vue thérapeutique est de 3,0 mg/jour.

4. Dérivé de carbamoyl-cyclohexane pour une utilisation selon la revendication 1 ou 2, **caractérisé en ce que** la quantité efficace du point de vue thérapeutique est de 4,5 mg/jour.

5. Dérivé de carbamoyl-cyclohexane pour une utilisation selon la revendication 1 ou 2, **caractérisé en ce que** la quantité efficace du point de vue thérapeutique est de 6 mg/jour.

6. Dérivé de carbamoyl-cyclohexane pour une utilisation selon la revendication 1 ou 2, **caractérisé en ce que** la quantité efficace du point de vue thérapeutique est de 9 mg/jour.

7. Dérivé de carbamoyl-cyclohexane pour une utilisation selon la revendication 1 ou 2, **caractérisé en ce que** la quantité efficace du point de vue thérapeutique est de 12 mg/jour.

8. Dérivé de carbamoyl-cyclohexane pour une utilisation selon l'une quelconque des revendications 1 à 7, **caractérisé en ce que** la quantité efficace du point de vue thérapeutique est divisée en une, deux, trois ou quatre doses quotidiennes.

9. Dérivé de carbamoyl-cyclohexane pour une utilisation selon l'une quelconque des revendications 1 à 8, **caractérisé en ce que** la manie aiguë est associée à un trouble bipolaire.

10. Dérivé de carbamoyl-cyclohexane pour une utilisation selon la revendication 9, **caractérisé en ce que** le trouble bipolaire est un trouble bipolaire I.

11. Dérivé de carbamoyl-cyclohexane pour une utilisation selon la revendication 9, **caractérisé en ce que** le trouble bipolaire est un trouble bipolaire II.

12. Dérivé de carbamoyl-cyclohexane pour une utilisation selon la revendication 9, **caractérisé en ce que** le trouble bipolaire est un trouble cyclothymique.

13. Dérivé de carbamoyl-cyclohexane pour une utilisation selon l'une quelconque des revendications 1 à 7, **caractérisé en ce que** la manie aiguë est associée à des épisodes maniaques aigus et mixtes.
